(19) Europäisches Patentamt
European Patent Office
Office européen des brevets

(11) EP 2 524 689 B1

(12) # EUROPEAN PATENT SPECIFICATION

(45) Date of publication and mention of the grant of the patent:
**05.10.2016 Bulletin 2016/40**

(21) Application number: **10841054.9**

(22) Date of filing: **28.12.2010**

(51) Int Cl.:
***A61K 9/20*** *(2006.01)*
***A61K 31/437*** *(2006.01)*
***A61K 31/4422*** *(2006.01)*
***A61K 31/445*** *(2006.01)*
***A61K 31/519*** *(2006.01)*
***A61K 47/32*** *(2006.01)*
***A61K 47/38*** *(2006.01)*

(86) International application number:
**PCT/JP2010/073763**

(87) International publication number:
**WO 2011/081199 (07.07.2011 Gazette 2011/27)**

(54) **Method for producing donepezil tablets**

Herstellungsverfahren von Donepezil Tabletten

Procédé de préparation de tablettes de donepezil

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2009 JP 2009298548**

(43) Date of publication of application:
**21.11.2012 Bulletin 2012/47**

(73) Proprietor: **Nipro Corporation**
**Osaka-shi, Osaka 531-8510 (JP)**

(72) Inventors:
- **KUNINOBU Kennichiro**
  **Osaka-shi**
  **Osaka 531-8510 (JP)**
- **HOASHI Yohei**
  **Osaka-shi**
  **Osaka 531-8510 (JP)**
- **KATAYAMA Naohisa**
  **Osaka-shi**
  **Osaka 531-8510 (JP)**
- **KAI Toshiya**
  **Osaka-shi**
  **Osaka 531-8510 (JP)**

(74) Representative: **Beckmann, Claus et al**
**Kraus & Weisert**
**Patentanwälte PartGmbB**
**Thomas-Wimmer-Ring 15**
**80539 München (DE)**

(56) References cited:
**WO-A1-2009/001146**
**WO-A2-2007/108010**
**JP-A- 2000 514 830**
**JP-A- 2002 012 541**
**JP-A- 2008 525 313**
**JP-A- 2009 114 113**
**JP-A- 2009 132 698**
**JP-B- 3 770 518**
**JP-B1- 4 257 865**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to a method for producing an oral preparation with which generation of an analogue resulting from a medicinal component is suppressed, the unpleasant taste such as bitterness resulting from the medicinal component, namely donepezil, is reduced, the dissolution behavior of the medicinal component is controlled, and which has reduced friability, and the present invention also relates to a production method therefor.

### Background Art

Some medicinal components contained in oral preparations are problematic in that they are partially lost as time passes due to the decomposition reaction or the like caused by, for example, light. Also, some medicinal components contained in oral preparations are problematic in that the recipient suffers when taking a preparation because some medicinal components have a bitter taste. Furthermore, some oral preparations and, in particular, solid preparations are problematic in that they have a high level of friability that allows, for example, a part of solid preparations to be chipped off by, for example, an external impact received after being shaped. Accordingly, it is difficult to use an automatic packaging machine at the drug dispensing site, resulting in problems such as significantly poor drug dispensing efficiency.

One example of medicinal components that have such problems is donepezil. Donepezil is a therapeutic agent for Alzheimer-type dementia and has a structural formula as shown in Formula 1 below.

[Formula 1]

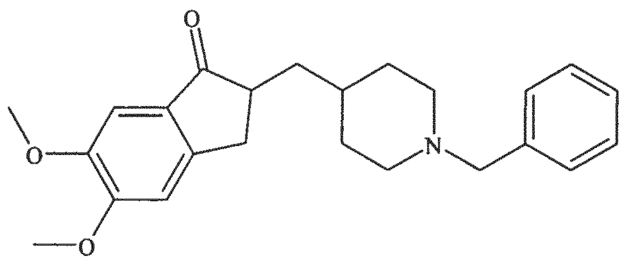

A tablet that contains donepezil hydrochloride as a medicinal component (hereinafter referred to as a donepezil hydrochloride-containing tablet) is already disclosed in Non-Patent Document 1. However, the donepezil hydrochloride-containing tablet described in Non-Patent Document 1 is problematic in that, in the case where it is stored for a long period of time, donepezil hydrochloride, which is a medicinal component unstable to light, is lost. Also, the donepezil hydrochloride-containing tablet described in Non-Patent Document 1 has a problem of a high level of friability.

Also, Patent Document 1 discloses an oral pharmaceutical composition containing an anionic high-molecular substance and a basic medicinal substance that has an unpleasant taste. Donepezil hydrochloride is described as one of the basic medicinal substances that have an unpleasant taste. Patent Document 1 describes, as the effect of the invention, that the unpleasant taste such as bitterness and numbness brought about by donepezil hydrochloride can be masked.

However, regarding the oral donepezil hydrochloride pharmaceutical composition disclosed in Patent Document 1, Patent Document 1 is silent as to the effect of the invention other than the unpleasant-taste masking effect.

Patent Document 2 describes a pharmaceutical composition for oral administration comprising donepezil or a pharmaceutically acceptable salt thereof and an effective amount of a pH-dependent excipient as a taste-masking agent to suppress the release of the donepezil in the pH environment of the oral cavity and increase the release of donepezil in an acidic environment.

### Prior Art Documents

#### Patent Documents

Patent Document 1: Japanese Laid-Open Patent Publication No. H11-228450
Patent Document 2: International Patent Application WO 2009/001146 A1

Non-Patent Documents

Non-Patent Document 1: Package insert of Aricept (registered trademark) D tablets

Summary of Invention

Problems to be Solved by the Invention

An object of the present invention is to provide a method for producing a donepezil hydrochloride-containing oral preparation having better qualities than donepezil hydrochloride-containing tablets produced by conventional techniques.

Specifically, for example, when the donepezil hydrochloride-containing tablet described in Non-Patent Document 1 is photo-irradiated at 300000 lux·hr, a donepezil analogue in the tablet is increased to about 0.73 % by weight, and accordingly an object is, for example, to make it possible to create a method for producing a novel donepezil hydrochloride-containing oral preparation with which this increase of the donepezil analogue can be suppressed at low cost, and in a simple manner and thus to provide a method for producing donepezil hydrochloride-containing oral preparation in which the donepezil hydrochloride content is not reduced over a longer period of time and which is safe for patients.

Means for Solving the Problems

The present invention provides the following:

(1) A method for producing an oral preparation comprising donepezil hydrochloride, according to claim 1. More specifically, the preparation is obtained through steps 1 to 6 below:

1. a step of obtaining a mixture containing donepezil hydrochloride and a first additive, wherein the first additive is a diluting agent selected from the group consisting of sugar alcohols, saccharides or starches,
2. a step of dissolving or suspending a binder in a granulation liquid preparation solvent to obtain a granulation liquid,
3. a step of adding the granulation liquid to the mixture and carrying out granulation with an agitation granulator to obtain granules,
4. a step of dissolving or suspending a coating agent, a lubricant, and/or a low-viscosity binder in a spray liquid preparation solvent to obtain a spray liquid,
5. a step of spraying the spray liquid onto the granules to coat the granules to obtain coated granules, and
6. a step of mixing the coated granules with a second additive and a disintegrator that has a carboxymethyl group, and tableting the mixture.

(2) The method according to (1), wherein the coating agent is an acrylic polymer-based coating agent.
(3) The method according to (1), wherein the coating agent is Eudragit NE.
(4) The method according to (1), wherein the disintegrator that has a carboxymethyl group is carmellose, carmellose sodium, carmellose calcium, croscarmellose sodium, sodium carboxymethyl starch, or carboxy methyl ethyl cellulose.

The present invention will now be described in detail below.

Donepezil herein refers to the compound represented by Formula 1 above. Donepezil suppresses decomposition of acetylcholine in the brain by inhibiting acetylcholinesterase. Due to this effect, donepezil exhibits a pharmacological effect of preventing a decrease of the activity of the cholinergic nervous system in the brain, and has been used as a therapeutic agent of Alzheimer-type dementia.

Donepezil is a medicinal substance that exhibits a strong bitter taste when administered, which makes it difficult to take.

Examples of further additives usable in the method of the present invention include binders, lubricants, disintegrators, plasticizers, antioxidants, antistatic agents, pH adjusters, fluidizers, surfactants, coloring agents, and the like.

Examples of the second additive usable in the method of the present invention include diluting agents, lubricants, disintegrators, antioxidants, antistatic agents, pH adjusters, fluidizers, surfactants, coloring agents, and the like.

Specific examples of sugar alcohols usable in the method of the present invention include mannitol, erythritol, xylitol, maltitol, sorbitol, and the like. More preferable are mannitol and xylitol, and most preferable is mannitol.

Specific examples of saccharides usable in the method of the present invention include hydrates, non-hydrates,

or the like of lactose, sucrose, saccharose, trehalose, fructose, glucose, and the like. More preferable are lactose and sucrose, and most preferable is lactose.

Specific examples of starches usable in the method of the present invention include corn starch, potato starch, and the like.

Specific examples of binders usable in the method of the present invention include sodium alginate, ethylcellulose, carrageenan, gelatin, hydroxypropylcellulose, polyvinylpyrrolidone, carboxy vinyl polymer, agar, copolyvidone, purified shellac, dextrin, hydroxyethyl cellulose, hydroxyethyl methyl cellulose, hydroxypropyl starch, hydroxypropyl cellulose, vinylpyrrolidone-vinyl acetate copolymer, hypromellose, partially pregelatinized starch, pullulan, pectin, polyvinyl alcohol-polyethylene glycol graft copolymer, povidone, polyvinyl alcohol, methacrylic acid copolymer L, methacrylic acid copolymer LD, methacrylic acid copolymer S, methylcellulose, and the like.

Examples of binders preferably used during the preparation of the granulation liquid of the present invention include hydroxypropyl cellulose, polyvinylpyrrolidone, hypromellose, and polyvinyl alcohol

Examples of lubricants used in the present invention include talc, titanium oxide, glycerine, glycerol fatty acid ester, wheat starch, sucrose fatty acid ester, stearyl alcohol, stearic acid and salts thereof, cetanol, gelatin, polyoxyethylene-polyoxypropylene glycols, polysorbates, macrogols, glyceryl monostearate, sodium lauryl sulfate, and the like.

Examples of lubricants preferably used during the preparation of the spray liquid of the present invention include magnesium stearate, talc, and titanium oxide.

Examples of disintegrators used in the present invention include corn starch, starch, crystalline cellulose, stearic acid and salts thereof talc, crospovidone, cellulose or derivatives thereof and the like.

Examples of disintegrator preferably used in the present invention include crystalline cellulose and corn starch.

Examples of plasticizers used in the present invention include triacetin, triethyl citrate, polypropylene glycol, polyethylene glycol, glycerine, polysorbate 80, diethyl sebacate, dibutyl sebacate, stearic acid, and the like.

Examples of antioxidants used in the present invention include tocopherol, ascorbic acid, sodium hydrogen sulfite, sodium sulfite, sodium edetate, erythorbic acid, cysteine hydrochloride, dried sodium sulfite, citric acid hydrate, dibutylhydroxytoluene, soybean lecithin, natural vitamin E, tocopherol, sodium pyrosulfite, butylhydroxyanisol, propyl gallate, and the like.

Examples of antistatic agents used in the present invention include hydrous silicon oxide, light anhydrous silicon, talc, and the like.

Examples of pH adjusters used in the present invention include citric acid and salts thereof phosphoric acid and salts thereof, carbonic acid and salts thereof, tartaric acid and salts thereof, fumaric acid and salts thereof, acetic acid and salts thereof, amino acids and salts thereof, succinic acid and salts thereof lactic acid and salts thereof, and the like.

Examples of fluidizers used in the present invention include light anhydrous silicic acid, hydrous silicon oxide, titanium oxide, talc, stearic acid and salts thereof heavy silicic acid anhydride, and the like.

Examples of fluidizers preferably used in the present invention include light anhydrous silicic acid, titanium oxide, talc, and the like.

Examples of surfactants used in the present invention include phospholipid, glycerol fatty acid ester, polyoxyethylene fatty acid ester, sorbitan fatty acid ester, polyethylene glycol fatty acid ester, polyoxyethylene hydrogenated castor oil, polyoxyethylene alkyl ether, sucrose fatty acid ester, sodium lauryl sulfate, polysorbates, sodium hydrogen phosphates, potassium hydrogen phosphates, and the like.

Examples of coloring agents used in the present invention include iron sesquioxide, yellow iron sesquioxide, tar-based color, aluminum chelate, titanium oxide, talc, and the like.

Examples of coloring agents preferably used in the present invention include titanium oxide, talc, iron sesquioxide, tar-based color, and the like.

Examples of solvents for the preparation of the granulation liquid usable in the method of the present invention include water, ethanol, isopropyl alcohol, and the like.

Specific examples of coating agents usable in the method of the present invention include ethyl acrylate-methyl methacrylate copolymer, aminoalkyl methacrylate copolymer, ethyl cellulose, carboxyvinyl polymer, methacrylic acid copolymer, dimethylaminoethyl methacrylate-methyl methacrylate copolymer, and the like.

Examples of coating agents preferably used in the present invention include acrylic polymerbased coating agents such as Eudragit NE30D and Eudragit L.

Specific examples of low-viscosity binders usable in the method of the present invention include sodium alginate, ethyl cellulose, carrageenan, gelatin, hydroxypropyl cellulose, polyvinylpyrrolidone, carboxyvinyl polymer, agar, copolyvidone, purified shellac, dextrin, hydroxyethyl cellulose, hydroxyethyl methyl cellulose, hydroxypropyl starch, hydroxypropyl cellulose, vinylpyrrolidone-vinyl acetate copolymer, hypromellose, partially pregelatinized starch, pullulan, pectin, polyvinyl alcohol-polyethylene glycol graft copolymer, povidone, polyvinyl alcohol, methacrylic acid copolymer L, methacrylic acid copolymer LD, methacrylic acid copolymer S, methylcellulose, and the like.

Examples of low-viscosity binders preferably used in the present invention include methylcellulose, hypromellose, and the like.

Specific examples of disintegrators that have a carboxymethyl group usable in the method of the present invention include carmellose, carmellose sodium, carmellose calcium, croscarmellose sodium, sodium carboxymethyl starch, carboxy methyl ethyl cellulose, and the like.

Examples of disintegrators that have a carboxymethyl group used in the present invention include carmellose, croscarnaellose sodium, carmellose calcium, and sodium carboxymethyl starch.

Examples of solvents for the preparation of the spray liquid usable in the method of the present invention include water, ethanol, and the like.

It is possible to carry out agitation and granulation in the present invention with a generally known agitation granulator. Examples of generally known agitation granulators include vertical granulators, high-speed mixers, and the like.

The tableting method for tablets to be produced by the present invention is not particularly limited as long as the effect of the present invention is demonstrated. Examples of the tableting method in the present invention include dry indirect compression method, wet indirect compression method, dry direct compression method, and the like.

Tablets produced by the present invention are shaped using, for example, a single-punch tableting machine, a rotary tableting press machine, or the like. The pressure for tableting is usually 4 to 20 $kN/cm^2$. The shape of the solid preparation produced by the present invention is not particularly limited, and specific examples include shapes such as round, caplet, doughnut and oblong as well as multilayered tablets and cored tablets. Moreover, the tablets may be provided, if necessary, with distinguishing characters, symbols, or marks and, moreover, may be provided with a line along which tablets can be broken.

The donepezil analogue, which can be reduced by the present invention, can be quantified as follows.

From a chromatogram obtained by HPLC, the sum of the peak areas excluding the main-peak area and the additive-derived peak areas is calculated, the resulting value is divided by the value of the main-peak, the obtained value is regarded as the amount of the analogue, and comparison/evaluation is carried out. More specifically, comparison/evaluation can be carried out using the test method described in paragraph [0055].

Effects of Invention

The present invention has made it possible to obtain a donepezil hydrochloride-containing tablet with which generation of a donepezil analogue in the tablet, which occurs through photo-irradiation for a long period of time, can be less than with conventional donepezil hydrochloride-containing tablets.

Also, the present invention has made it possible to reduce an unpleasant taste such as bitterness resulting from donepezil hydrochloride felt after taking a donepezil hydrochloride-containing tablet in a sensory test.

Also, the present invention has made it possible to obtain a donepezil hydrochloride-containing tablet with which the dissolution behavior of donepezil hydrochloride immediately after taking the donepezil hydrochloride-containing tablet can be more controlled compared with conventional donepezil hydrochloride-containing tablets.

Also, the present invention has made it possible to obtain a donepezil hydrochloride-containing tablet having a lower level of friability than conventional donepezil hydrochloride-containing tablets.

Also, the present invention has made it possible to obtain a donepezil hydrochloride-containing tablet having a lower level of porosity.

Mode for Carrying Out the Invention

The best mode for carrying out the invention will now be disclosed below. Examples

Example 1:

After 65 parts by weight of donepezil hydrochloride, and 110 parts by weight of D-mannitol were mixed to give a mixture, the mixture was introduced into an agitation granulator (VG-05, manufactured by Powrex Corporation), and a granulation liquid composed of 2 parts by weight of hypromellose and 20 parts by weight of purified water was added, to give granules. The granules were dried in a fluidized-bed drier (multiplex MP-01, manufactured by Powrex Corporation), and pulverization and particle size regulation were carried out with a power mill. The dried granules were sprayed with a spray liquid composed of 150 parts by weight of Eudragit NE30D, 25 parts by weight of talc, 25 parts by weight of titanium oxide, 10 parts by weight of methylcellulose, and 200 parts by weight of purified water to give coated granules. Two parts by weight of light anhydrous silicic acid, 10 parts by weight of carmellose, 2 parts by weight of magnesium stearate, 10 parts by weight of corn starch, 5 parts by weight of crystalline cellulose, and 101 parts by weight of D-mannitol were added to and mixed with 20 parts by weight of the prepared coated granules, and tableting was carried out with a rotary tableting machine (VIRGO, manufactured by Kikusui Seisakusho Ltd.).

Example 2:

Two parts by weight of light anhydrous silicic acid, 10 parts by weight of carmellose calcium, 2 parts by weight of magnesium stearate, 10 parts by weight of corn starch, 5 parts by weight of crystalline cellulose, and 101 parts by weight of D-mannitol were added to and mixed with 20 parts by weight of the coated granules as prepared in Example 1, and tableting was carried out with a rotary tableting machine (VIRGO, manufactured by Kikusui Seisakusho Ltd.).

Example 3:

Ten parts by weight of sodium carboxymethyl starch, 2 parts by weight of light anhydrous silicic acid, 2 parts by weight of magnesium stearate, 10 parts by weight of corn starch, 5 parts by weight of crystalline cellulose, and 101 parts by weight of D-mannitol were added to and mixed with 20 parts by weight of the coated granules as prepared in Example 1, and tableting was carried out with a rotary tableting machine (VIRGO, manufactured by Kikusui Seisakusho Ltd.).

Example 4:

After 65 parts by weight of donepezil hydrochloride and 110 parts by weight of D-mannitol were mixed to give a mixture, the mixture was introduced into an agitation granulator (high-speed mixer FS-02, manufactured by Fukae Seisakusho), and a granulation liquid composed of 2 parts by weight of hydroxypropyl cellulose and 20 parts by weight of purified water was added, to give granules. The granules were dried in a fluidized-bed drier (Multiplex MP-01, manufactured by Powrex Corporation), and pulverization and particle size regulation were carried out with a power mill. The dried granules were sprayed with a spray liquid composed of 150 parts by weight of Eudragit NE30D, 50 parts by weight of titanium oxide, 10 parts by weight of hypromellose, and 200 parts by weight of purified water to give coated granules. Two parts by weight of light anhydrous silicic acid, 10 parts by weight of carmellose, 2 parts by weight of magnesium stearate, 10 parts by weight of corn starch, 5 parts by weight of crystalline cellulose, and 101 parts by weight of D-mannitol were added to and mixed with 20 parts by weight of the prepared coated granules, and tableting was carried out with a rotary tableting machine (VIRGO, manufactured by Kikusui Seisakusho Ltd.).

Example 5 (for comparison and/or reference only):

After 50 parts by weight of zolpidem tartrate and 50 parts by weight of lactose hydrate were mixed to give a mixture, the mixture was introduced into an agitation granulator (VG-05, manufactured by Powrex Corporation), and a granulation liquid composed of 2 parts by weight of methylcellulose and 20 parts by weight of purified water was added, to give granules. The granules were dried in a fluidized-bed drier (Multiplex MP-01, manufactured by Powrex Corporation), and pulverization and particle size regulation were carried out with a power mill. The dried granules were sprayed with a spray liquid composed of 150 parts by weight of Eudragit L30D55, 50 parts by weight of titanium oxide, 5 parts by weight of triethyl citrate, and 200 parts by weight of purified water to give coated granules. Thirty parts by weight of croscarmellose sodium, 2 parts by weight of magnesium stearate, and 90 parts by weight of D-mannitol were added to and mixed with 30 parts by weight of the prepared coated granules, and tableting was carried out with a rotary tableting machine (VIRGO, manufactured by Kikusui Seisakusho Ltd.).

Comparative Example 1:

Ten parts by weight of crospovidone, 2 parts by weight of light anhydrous silicic acid, 2 parts by weight of magnesium stearate, 10 parts by weight of corn starch, 5 parts by weight of crystalline cellulose, and 101 parts by weight of D-mannitol were added to and mixed with 20 parts by weight of the coated granules as prepared in Example 1, and tableting was carried out with a rotary tableting machine (VIRGO, manufactured by Kikusui Seisakusho Ltd.).

Comparative Example 2:

Ten parts by weight of crystalline cellulose, 2 parts by weight of magnesium stearate, and 118 parts by weight of D-mannitol were added to and mixed with 20 parts by weight of the coated granules as prepared in Example 1, and tableting was carried out with a rotary tableting machine (VIRGO, manufactured by Kikusui Seisakusho Ltd.).

Comparative Example 3:

Two parts by weight of magnesium stearate, and 128 parts by weight of F-Melt Type C were added to and

mixed with 20 parts by weight of the coated granules as prepared in Example 1, and tableting was carried out with a rotary tableting machine (VIRGO, manufactured by Kikusui Seisakusho Ltd.).

Comparative Example 4:

After 65 parts by weight of donepezil hydrochloride and 110 parts by weight of D-mannitol were mixed to give a mixture, the mixture was introduced into a fluidized-bed drier granulator (Multiplex MEP-01, manufactured by Powrex Corporation), and a granulation liquid composed of 3 parts by weight of hydroxypropyl cellulose and 50 parts by weight of purified water was added, to give granules. Next, the granules were subjected to pulverization and particle size regulation in a power mill. The granules after pulverization and particle size regulation were sprayed with a spray liquid composed of 150 parts by weight of Eudragit NE30D, 50 parts by weight of talc, and 200 parts by weight of purified water to give coated granules. Two parts by weight of light anhydrous silicic acid, 10 parts by weight of carmellose, 2 parts by weight of magnesium stearate, 10 parts by weight of corn starch, 5 parts by weight of crystalline cellulose, and 101 parts by weight of D-mannitol were added to and mixed with 20 parts by weight of the prepared coated granules, and tableting was carried out with a rotary tableting machine (VIRGO, manufactured by Kikusui Seisakusho Ltd.).

Comparative Example 5:

Thirty parts by weight of carmellose sodium, 2 parts by weight of magnesium stearate, and 98 parts by weight of D-mannitol were added to and mixed with 20 parts by weight of the dried granules as prepared in Example 5, and tableting was carried out with a rotary tableting machine (VIRGO, manufactured by Kikusui Seisakusho Ltd.).

Comparative Example 6:

Thirty parts by weight of carmellose, 5 parts by weight of magnesium stearate, and 155 parts by weight of D-mannitol were added to and mixed with 30 parts by weight of the dried granules as prepared in Example 4, and tableting was carried out with a rotary tableting machine (VIRGO, manufactured by Kikusui Seisakusho Ltd.).

Comparative Example 7:

Aricept (registered trademark) 10 mg D tablets (produced and distributed by Eisai Co., Ltd.) were used.

Test Example 1: Test for measurement of analogue content after long-term storage under photo-irradiated conditions

The donepezil hydrochloride-containing oral preparations obtained in Examples 1 to 4 and Comparative Example 7 were each placed in a photostability test chamber (LTB-180C type, manufactured by Nagano Science Co., Ltd.), and irradiated at an illuminance of 1000 lux/hr under a D65 lamp so as to attain a cumulative illuminance of 300000 lux/hr. Thereafter, each sample was dissolved in a solution for use as a mobile phase such that the concentration of donepezil hydrochloride was 1 mg/mL. The used mobile phase was a solution prepared by mixing an aqueous 1-decanesulfonic acid solution and acetonitrile and perchloric acid in a ratio of 1300 : 700 : 1. The peak area ratio of analogue to donepezil hydrochloride was calculated using a test instrument (high-speed liquid chromatograph: LC-20A, manufactured by Shimadzu Corporation) and a UV detector as a detector while maintaining the temperature of a column (Mightsil ODS RP-18 GP 150-4.6), into which a sample was introduced, at 40°C under conditions of the amount of sample introduced into the column of 10 μL, the flow rate of 1.3 mL/min, and the measurement wavelength of 271 nm to quantify the analogue. Test results are shown in Table 1.

[Table 1]

| | Analogue Content (% by weight) |
|---|---|
| Example 1 | 0.36 |
| Example 2 | 0.32 |
| Example 3 | 0.32 |
| Example 4 | 0.25 |
| Comparative Example 7 | 0.73 |

It is clear from these results that, with donepezil hydrochloride-containing tablets of the present invention, the analogue content can be lower than that with a conventional donepezil hydrochloride-containing tablet.

Test Example 2: Sensory test of oral preparation

The taste of a tablet until it completely dissolved in saliva in the mouth of healthy male adults (27 years old, 175 cm height, 65 kg body weight) was evaluated. The test was carried out twice, and results of comprehensive evaluation of the test that was carried out twice are shown in Table 2.

-: Absolutely no unpleasant taste or bitterness was felt.
±: Slight unpleasant taste or bitterness was felt.
+: Unpleasant taste or bitterness was felt.
++: Considerable unpleasant taste or bitterness was felt.

[Table 2]

| | Evaluation of Bitterness | Disintegration Time (sec.) |
|---|---|---|
| Example 1 | ± | 20 |
| Example 2 | - | 18 |
| Example 3 | - | 21 |
| Example 4 | ± | 23 |
| Example 5 (*) | - | 24 |
| Comparative Example 1 | + | 20 |
| Comparative Example 2 | ++ | 25 |
| Comparative Example 3 | + | 30 |
| Comparative Example 4 | + | 29 |
| Comparative Example 5 | ++ | 112 |
| Comparative Example 6 | + | 30 |
| Comparative Example 7 | + | 20 |
| (*) for comparison and/or reference only | | |

It is clear from these results that an unpleasant taste such as bitterness resulting from the medicinal component donepezil hydrochloride is reduced in oral preparations obtained by the method of the present invention.

Test Example 3: Dissolution test of oral preparation

Three tablets prepared in each of Examples 1 to 3 and Comparative Examples 1 to 3 were subjected to a comparison of the dissolution rate (%) of the medicinal component by the upright-inverted syringe method. Values measured 30 seconds after the beginning of the test were regarded as dissolution rates and shown in Table 3.

The "upright-inverted syringe method" herein uses a simple dissolution test method (the upright-inverted syringe method) of Nakamura et al. that mimics the oral cavity This method enables a measurement of the amount of a drug dissolved and an evaluation of bitter taste masking in comparison with a threshold of feeling a bitter taste (reference: Yasuhiko Nakamura et al., "Ryuushi Sekkei To Seizai Gijyutsu (Particle Designing and Formulation Technique)" 121-128 (1993)). The upright-inverted syringe method herein is carried out as follows.

(Test on donepezil hydrochloride-containing particles)

Provide a 10 mL plastic syringe (manufactured by Nipro) to the end of which a 25 mm size filter having a pore size of 0.22 μm to 0.45 μm (Ekicrodisc manufactured by Pall Corporation) is attached, with the fluid outlet at the end of the filter being wrapped and blocked with a thermoplastic film (PARAFILM (registered trademark) M) to prevent fluid leakage. Introduce into this 10 mL glass syringe a tablet corresponding to 15 mg of donepezil hydrochloride and 10 mL

of water maintained at 37 ± 1°C. Attach a plunger at the same time, turn the syringe into an upright or inverted position at a rate of every 3 seconds for 30 seconds for a total of 10 times so that the PARAFILM detaches and filtration is carried out with the filter; discard the first 5 mL of the nitrate; recover the remaining filtrate; and measure the amount of donepezil hydrochloride.

Measurement of the amount of donepezil hydrochloride can be carried as follows.

The recovered filtrate is used as a test solution. Separately, about 150 mg of a donepezil hydrochloride reference standard that has been dried at 105°C for 2 hours under reduced pressure is precisely weighed out, water as used for dissolution in this test is added, ultrasonic irradiation is carried out to dissolve the reference standard, and water is further added to attain 100 mL precisely, thus giving a standard solution. Using this standard solution and test solution and a UV-Vis absorption spectrometer (U-3300, manufactured by Hitachi, Ltd.) as a detector, 4 mL of a sample is introduced into a 10 mm × 10 mm quartz cell, and spectrophotometry is carried out at a measurement wavelength of 271 nm. Using the numerical value of the detected donepezil hydrochloride, the amount of dissolution by a simple dissolution test (the syringe upright-inverted method) is calculated by Equation (1) below. The obtained test results are shown in Table 3.

$$\text{Rate (\%) of dissolution by upright-inverted syringe method} = Ws \times At/As \times 2/3 \quad (1)$$

where

Ws: Amount of donepezil hydrochloride reference standard (mg)

At: Absorbance of test solution

As: Absorbance of standard solution

[Table 3]

| | Dissolution Rate (%) |
|---|---|
| Example 1 | 22 |
| Example 2 | 7 |
| Example 3 | 16 |
| Comparative Example 1 | 54 |
| Comparative Example 2 | 53 |
| Comparative Example 3 | 49 |

It is clear from these results that with donepezil hydrochloride-containing tablets of the present invention, the dissolution behavior of donepezil hydrochloride immediately after administration can be controlled.

Test Example 4: Test for measurement of friability of oral preparations

The donepezil hydrochloride-containing oral preparations obtained in Examples 1, 4, and 5 and Comparative Example 7 were subjected to a test in accordance with a tablet friability testing method (The Japanese Pharmacopoeia Fifteenth Edition) to compare their level of friability (%). The results are shown in Table 4.

[Table 4]

| | Level of Friability (%) |
|---|---|
| Example 1 | 0.07 |
| Example 4 | 0.08 |

(continued)

| | Level of Friability (%) |
|---|---|
| Example 5 (*) | 0.08 |
| Comparative Example 7 | 0.34 |
| (*) for comparison and/or reference only | |

These results made it clear that oral preparations obtained by the method of the present invention have a lower level of friability than a conventional oral preparation.

Test Example 5: Test for determination of porosity of oral preparations

Enlarged images of granules and coated granules obtained in both Example 4 and Comparative Example 4 are presented in Figs. 1 to 4 below.

These results made it clear that the donepezil hydrochloride-containing tablets of the present invention have reduced porosity.

Industrial Applicability

The present invention has made it possible to provide a novel oral preparation that has better qualities from a number of different perspectives compared with conventional oral preparations having an unpleasant taste. Accordingly, it has become possible to provide an oral preparation having an unpleasant taste that is safer to patients who receive the preparation.

## Claims

1. A method for producing an oral preparation which comprises:

   (a) obtaining granules through agitation granulation of a mixture containing donepezil hydrochloride and a first additive with a granulation liquid containing a binder by using an agitation granulator, wherein the first additive is a diluting agent selected from the group consisting of sugar alcohols, saccharides or starches;
   (b) coating the granules by spraying a spray liquid onto the granules, which spray liquid is obtained by dissolving or suspending a coating agent, a lubricant and/or a low-viscosity binder in a spray liquid preparation solvent, to obtain coated granules; and
   (c) tableting a mixture containing the coated granules, a second additive and a disintegrator having a carboxymethyl group.

2. The method according to claim 1, wherein the coating agent is an acrylic polymer-based coating agent.

3. The method according to claim 1, wherein the disintegrator is carmellose, carmellose sodium, carmellose calcium, croscarmellose sodium, sodium carboxymethyl starch, or carboxy methyl ethyl cellulose.

## Patentansprüche

1. Verfahren zur Herstellung einer oralen Zubereitung, umfassend:

   (a) Herstellen eines Granulats mit Hilfe von Rührgranulation einer Mischung, enthaltend Donepezilhydrochlorid und einen ersten Zusatzstoff, mit einer Granulationsflüssigkeit, enthaltend ein Bindemittel, unter Verwendung eines Rührgranulators, wobei der erste Zusatzstoff ein Verdünnungsmittel, ausgewählt aus der Gruppe, bestehend aus Zuckeralkoholen, Sacchariden oder Stärken, ist;
   (b) Beschichten des Granulats durch Sprühen einer Sprühflüssigkeit auf das Granulat, wobei die Sprühflüssigkeit hergestellt wird durch Lösen oder Suspendieren eines Beschichtungsmittels, eines Schmiermittels und/oder eines Bindemittels mit niedriger Viskosität in einem Lösungsmittel zum Herstellen einer Sprühflüssigkeit, um ein beschichtetes Granulat zu erhalten; und

(c) Tablettieren einer Mischung, enthaltend das beschichtete Granulat, einen zweiten Zusatzstoff und ein Springmittel mit einer Carboxymethylgruppe.

**2.** Das Verfahren gemäß Anspruch 1, wobei das Beschichtungsmittel ein auf einem Acrylpolymer basiertes Beschichtungsmittel ist.

**3.** Das Verfahren gemäß Anspruch 1, wobei das Springmittel Carmellose, Natriumcarmellose, Calciumcarmellose, Natriumcroscarmellose, Natriumcarboxymethylstärke oder Carboxymethylethylcellulose ist.

**Revendications**

**1.** Procédé pour produire une préparation orale qui comprend :

(a) l'obtention de granules par le biais d'une granulation par agitation d'un mélange contenant de l'hydrochlorure de donepezil et un premier additif avec un liquide de granulation contenant un liant en utilisant un granulateur par agitation, dans lequel le premier additif est un agent de dilution sélectionné dans le groupe constitué d'alcools de sucre, de saccharides ou d'amidons ;
(b) l'enrobage des granules en pulvérisant un liquide de pulvérisation sur les granules, lequel liquide de pulvérisation est obtenu par la dissolution ou la suspension d'un agent d'enrobage, d'un lubrifiant et/ou d'un liant à faible viscosité dans un solvant de préparation de liquide de pulvérisation, afin d'obtenir des granules enrobées ; et
(c) la mise en tablettes d'un mélange contenant les granules enrobées, un second additif et un désintégrateur ayant un groupe carboxyméthyle.

**2.** Procédé selon la revendication 1, dans lequel l'agent d'enrobage est un agent d'enrobage à base de polymère acrylique.

**3.** Procédé selon la revendication 1, dans lequel le désintégrateur est de la carmellose, du sodium de carmellose, du calcium de carmellose, du sodium de croscarmellose, de l'amidon carboxyméthyle de sodium ou de la cellulose de carboxyméthyléthyle.

[Fig.1]

The granules obtained in Example 4

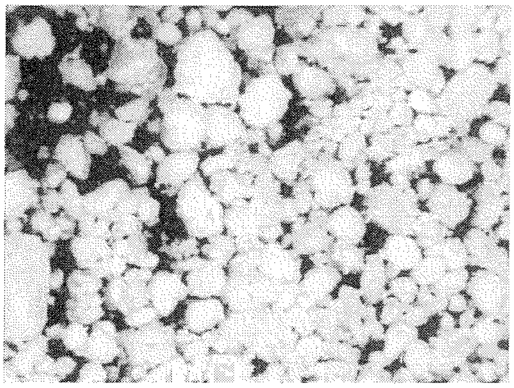

[Fig.2]

The coated granules obtained in Example 4

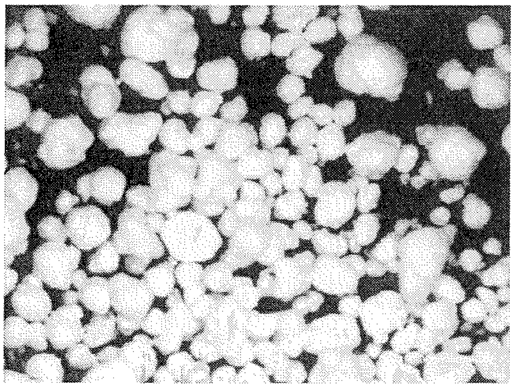

[Fig.3]

The granules obtained in Comparative Example 4

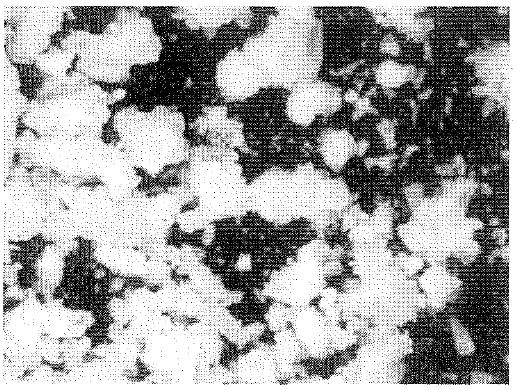

[Fig.4]

The coated granules obtained in Comparative Example 4

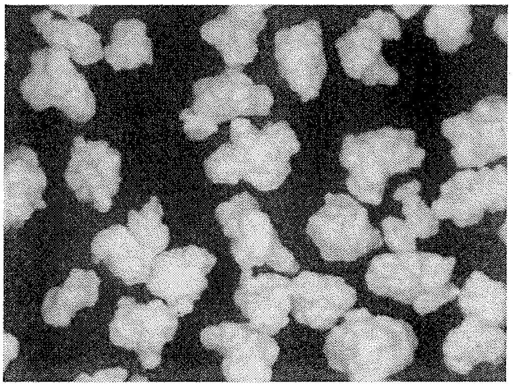

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11228450 B
- WO 2009001146 A1


### Non-patent literature cited in the description


- **YASUHIKO NAKAMURA et al.** *Ryuushi Sekkei To Seizai Gijyutsu (Particle Designing and Formulation Technique),* 1993, 121-128